# EUROPEAN PATENT APPLICATION

(11) **EP 2 412 354 A1**
(43) Date of publication of application: **01.02.2012**
(21) Application number: 10756000.5
(22) Date of filing: 19.03.2010
(51) Int. Cl.: A61F 13/496

(54) **UNDERWEAR-TYPE ABSORBENT ARTICLE**

(30) Priority: 23.03.2009 JP 2009071025; 23.03.2009 JP 2009071026
(71) Applicant: Kao Corporation, Chuo-ku Tokyo 103-8210 (JP)
(72) Inventor: SASAKI, Jun, Haga-gun Tochigi 321-3497 (JP); SATO, Takayuki, Haga-gun Tochigi 321-3497 (JP)
(74) Representative: Vossius & Partner
(86) International application number: PCT/JP2010/054798
(87) International publication number: WO 2010/110203

(57) **Abstract**

A disposable pull-on diaper (1) having both side edge portions (6a) of the front section and both side edge portions (6b) of the rear section overlapped together, and the overlapped portion (7) fusion bonded to form a pair of side seals (8). The overlapped portion (7) includes a waist opening-side waist end region (71), where a larger number of sheets are stacked than in other regions (72 and 73). The stacked sheet structure of the waist end region (71) in each of the front and rear sections extends between the overlapped portions in each of the front and rear sections. The side seal (8) has a smaller total length of fusion bonds (81) per unit length in its longitudinal direction measured along its skin side edge (8e) in its seal region (8a1) formed by fusion bonding the waist end region (71) than in the other seal regions (8a2 and 8a3) formed by fusion bonding the other regions (72 and 73) below the region (71).

## Description

### Technical Field

The present invention relates to a pull-on absorbent article, such as a disposal diaper.

### Background Art

A pull-on (or pant-type) absorbent article is known which includes a topsheet, a backsheet, and a liquid retentive absorbent member and has a front section and a rear section, with both lateral edges of the front section and both lateral edges of the rear section being overlapped face-to-face and fusion bonded to make a pair of side seals.
Various proposals have been made on the pattern of fusion bonds to be formed in the side seals for various purposes.
A pull-on (or pant-type) absorbent article is known which includes a topsheet, a backsheet, and a liquid retentive absorbent member and has a front section and a rear section, with both lateral edges of the front section and both lateral edges of the rear section being overlapped face-to-face and fusion bonded to make a pair of side seals.
Various proposals have been made on the pattern of fusion bonds to be formed in the side seals for various purposes.

For example, Applicant of the present invention previously proposed a disposable pull-on diaper providing a good fit while retaining good wearer comfort and texture (see patent literature 1 below), in which the parts of the side seal that are located in the waist opening peripheral portion and the leg opening peripheral portion have interrupted fusion bonds each having a non-fusion-bonded region in the middle thereof in the width direction of the side seal.

Patent literature 2 (see below) discloses a garment, such as a diaper, formed by overlapping front region seam panels and rear region seam panels to make a waist opening and two leg openings, in which the seam panels are overlapped by forming a number of bonds grouped in a plurality of clusters.

Applicant of the present invention also proposed a pull-on absorbent article of which the side seals have sufficient bonding strength, good texture, and ease of tear (see patent literature 3 below). The side seal is made up of strongly bonded regions formed by pressing from both the front section side and the rear section side, weakly bonded regions formed by pressing from either the front section side or the rear section side, and non-bonded regions having received no pressing force from either side.

### Citation List

### Patent Literature:

Patent Literature 1: JP 10-1372877A
Patent Literature 2: JP 2002-369842A
Patent Literature 3: JP 2001-120595A

### Summary of Invention

However, the disposable diaper of patent literature 1 can have the fusion bonds along the skin side edge of the side seal giving discomfort to the waist of a wearer or irritating the skin, e.g., of the waist, hands or legs when the diaper is pulled on by a wearer or a diaperer.
While a wearer wears a diaper, the waist circumference of the wearer is relatively large and changes with the change of wearer's posture or breathing. Therefore, cushioning and softness of the part of the diaper that is in contact with the waist are largely influential on the wearer comfort, In this regard, the garment of patent literature 2 lacks considerations for the softness and feel of the waist portion. Moreover, the seam panels of the front region and the seam panels of the back regions are overlapped with the exterior (or interior) side of the former and the interior (or exterior) side of the latter facing each other. This manner of overlapping needs complicated production equipment.
In the case of the disposable diaper of patent literature 3, the strongly bonded regions can give a wearer discomfort or irritate the skin of a wearer or a diaperer when a downward or sideways (in a direction vertical to the wearer's skin) pressing force is exerted to the waist opening peripheral portion in putting on the diaper or putting the diaper on or during wear.

The invention provides a pull-on absorbent article including a front section having lateral side edge portions and a rear section having lateral side edge portions, the lateral side edge portions of the front section and the lateral side edge portions of the rear section being overlapped to form overlapped portions, and the overlapped portions being fusion bonded to form a pair of side seals.
The overlapped portions each have a waist end region on a side of a waist opening. The waist end region includes a stack of larger number of sheets than any other region of the overlapped portion. The sheet stacked structure forming the waist end region in both the front section and the rear section extends over a whole distance between opposing overlapped portions of the front section and between opposing overlapped portions of the rear section, respectively. The side seal has a smaller total length of fusion bonds per unit length in its longitudinal direction measured along a skin side edge thereof in a seal region formed by fusion bonding the waist end region than in the other seal regions formed by fusion bonding an other regions below the waist end region.

The invention also provides a pull-on absorbent article including a front section having lateral side edge portions and a rear section having lateral side edge portions, the lateral side edge portions of the front section and the lateral side edge portions of the rear section being overlapped to form overlapped portions, and the overlapped portions being fusion bonded to form a pair of side seals.
The side seals each have a front side seal pattern formed by pressing a side of the front section and a rear side seal pattern formed by pressing a side of the rear section and includes an end region on the side of the waist opening and the other region. The front side seal pattern and the rear side seal pattern are different from each other. The front side seal pattern or the rear side seal pattern is different between the end region and the other region. The side seal has a lower fusion bond strength in the end region than in the other region.

### Brief Description of Drawings

[Fig. 1] FIG. 1 is a perspective of a disposable pull-on diaper according to a first embodiment of the invention.
[Fig. 2] FIG 2 is a plan of the disposable pull-on diaper of Fig. 1 in its flat-out, uncontracted state. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 3] FIG 3(a) is an enlarged cross-section taken along Y1-Y1 line in Fig. 2, FIG 3(b) is an enlarged cross-section taken along Y2-Y2 line in Fig. 2, and FIG 3(c) is an enlarged plan of a side seal.
[Fig. 4] FIG 4 is an enlarged plan of a side seal incorporating a modified embodiment of the invention (equivalent to Fig. 3(c)).
[Fig. 5] FIG 5 is an enlarged plan of a side seal incorporating another modified embodiment of the invention (equivalent to Fig. 3(c)).
[Fig. 6] FIG 6 is a schematic view of an embossing roller used to form side seals of the disposable pull-on diaper shown in Fig. 1.
[Fig. 7] FIG 7 is an enlarged plan of a side seal incorporating still another modified embodiment of the invention.
[Fig. 8] FIG 8 is an enlarged plan of a side seal incorporating still another modified embodiment of the invention.
[Fig. 9] FIG 9 is an enlarged plan of a side seal incorporating still another modified embodiment of the invention.
[Fig. 10] FIG 10 is a perspective of a disposable pull-on diaper according to a second embodiment of the invention.
[Fig. 11] FIG 11(a) is a cross-section equivalent to Fig. 3 (a) of the disposable pull-on diaper shown in Fig. 10, and FIG. 11(b) is a cross-section equivalent to Fig. 3(b) of the disposable pull-on diaper shown in Fig. 10.

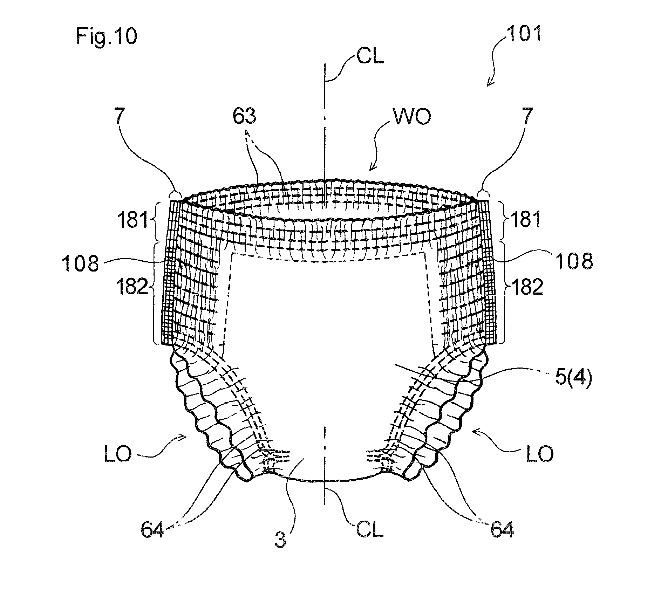

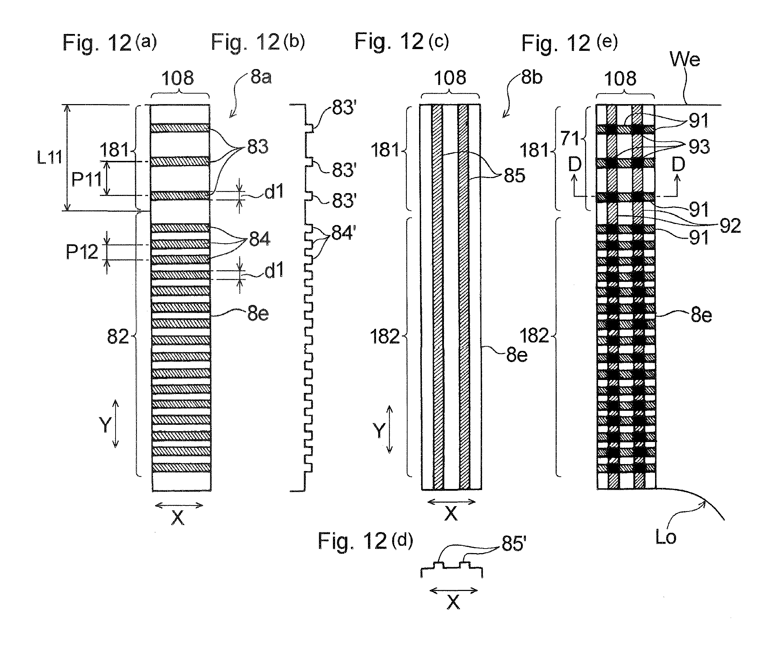

[Fig. 12] FIG. 12(a) shows the front side seal pattern of the side seal of the disposable pull-on diaper shown in Fig. 10; FIG 12(b) schematically illustrates the projections on a sealing roller used to form the front side seal pattern of FIG 12(a); FIG 12(c) shows the rear side seal pattern of the disposable pull-on diaper shown in Fig. 10; FIG 12(d) schematically illustrates the projections on a sealing roller used to form the front side seal pattern of FIG 12(c); and FIG. 12(e) illustrates the distribution of strongly bonded regions and weakly bonded regions in the side seal of the disposable pull-on diaper shown in Fig. 10.
[Fig. 13] FIG 13 is a cross-section taken along line D-D in Fig. 12(e).
[Fig. 14] FIG 14 is an enlarged cross-section of the pressure application part of sealing rollers used to form side seals.
[Fig. 15] FIG 15(a) shows a front side seal pattern or a rear side seal pattern of a disposable pull-on diaper according to a modified embodiment of the invention; FIG 15(b) shows a rear side seal pattern or a front side seal pattern of the disposable pull-on diaper; and FIG 15(c) illustrates the distribution of strongly bonded regions in the side seal of the disposable pull-on diaper.
[Fig. 16] FIG 16(a) shows a front side seal pattern or a rear side seal pattern of a disposable pull-on diaper according to a modified embodiment of the invention; FIG 16(b) shows a rear side seal pattern or a rear side seal pattern of the disposable pull-on diaper; and FIG 16(c) illustrates the distribution of strongly bonded regions in the side seal of the disposable pull-on diaper.

### Description of Embodiments

The pull-on absorbent article of the invention will be described based on its preferred embodiments.
A pull-on diaper according to a first embodiment of the invention (hereinafter also referred to as a diaper 1) has a pair of side seals 8 and 8. The side seals are formed by overlapping both lateral side edge portions 6a and 6a of the front section A and both lateral side edge portions 6b and 6b of the rear section B to make a pair of overlapped portions 7 and 7 and fusion bonding the overlapped portions 7 and 7. Each overlapped portion 7 is formed by putting the front section A and the rear section B together with their skin facing side inside and is oblong in the same direction as the longitudinal direction (vertical direction) of the diaper 1 while worn. The front section A, the rear section B, and the crotch section C are a portion applied to the front side of a wearer, to the rear side of a wearer, and to the crotch of a wearer, respectively, while the diaper 1 is worn.

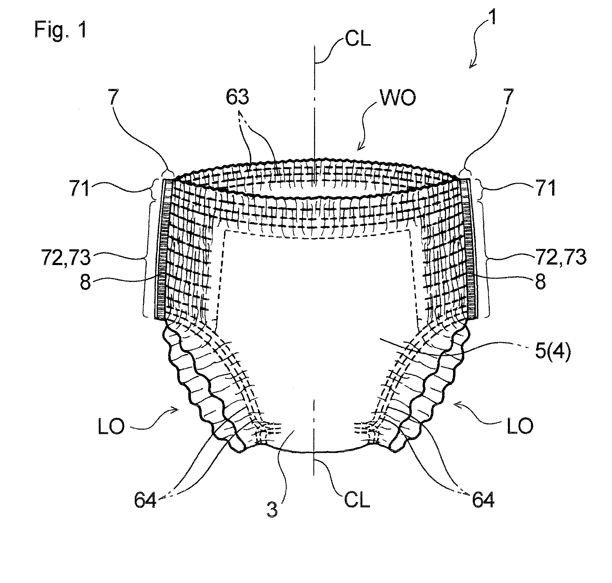

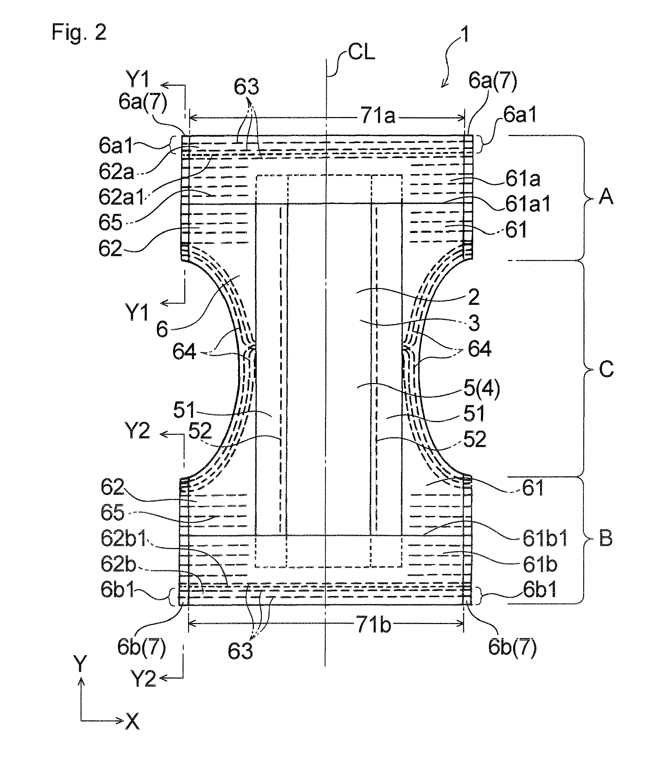

As shown in Figs. 1 and 2, the disposable diaper 1 is symmetrical about a centerline CL extending in its longitudinal direction (the direction from the front section A through the crotch section C to the rear section B). Therefore, while the diaper 1 will be described chiefly with reference to the structure on the left-hand side of Figs. 1 and 2, the description about the left-hand side applies to the right-hand side, except that they are of bilaterally symmetrical configuration. The longitudinal direction of the diaper 1 (the direction parallel to the centerline CL of the diaper 1 in its flat-out, uncontracted state) will hereinafter be referred to as the Y-direction.

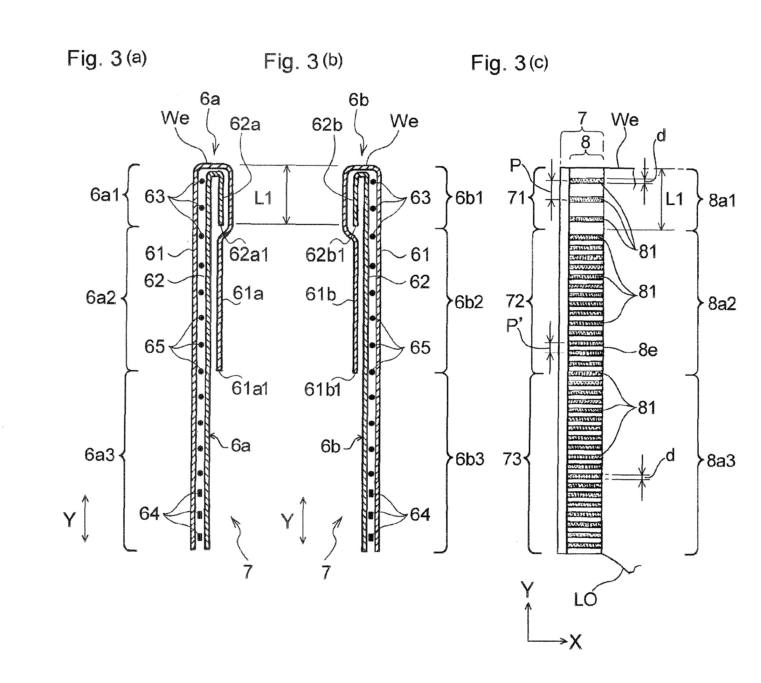

The overlapped portion 7 includes a waist end region 71 on the side of the waist opening WO and other regions 72 and 73. The waist end region 71 contains a larger number of sheets than the other regions 72 and 73. The stack of the sheets forming the waist end region 71 in both the front section A and the rear section B extends over the whole distance between opposing overlapped portions 71a of the front section A and between opposing overlapped portions 71b of the rear section B. The side seal 8 includes a seal region 8a1 formed by fusion bonding the waist end region 71 and seal regions 8a2 and 8a3 that are formed by fusion bonding the other regions 72 and 73 below the waist end region 71. The side seal (8) has a smaller total length of fusion bonds (81) per unit length in its longitudinal direction measured along the skin side edge (8e) thereof in its seal region (8a1) than in the seal regions (8a2) and (8a3) formed by fusion bonding the other regions (72 and 73) below the region (71) (see Fig. 3(c)).

The diaper 1 of the present embodiment will be described in greater detail.
As shown in Fig. 2, the diaper 1 of the present embodiment includes a substantially longitudinally oblong absorbent assembly 5 and an outer cover 6. The absorbent assembly 5 has a liquid permeable topsheet 2, a liquid impermeable or water repellent backsheet 3, and a liquid retentive absorbent member 4 interposed between the topsheet 2 and the backsheet 3. The outer cover 6 is located on the backsheet side (garment facing side) of the absorbent assembly 5 to fix the absorbent assembly 5.

The diaper 1 has both the lateral side edge portions 6a and 6a of its front section A and both the lateral side edge portions 6b and 6b of its rear section B overlapped to each other to make the overlapped portions 7 and 7 and has the overlapped portions fusion-bonded to form a pair of side seals 8 and 8, thereby taking on the configuration of pants having a waist opening WO and a pair of leg openings LO and LO.

The outer cover 6 extends outward from all the perimeter of the absorbent assembly 5 and has an outer sheet 61 that defines the exterior surface of the diaper and an inner sheet 62 overlaid on the inner side of the outer sheet 61. The outer sheet 61 and the inner sheet 62 are folded over along a line located outward of the longitudinal (Y-directional) ends of the absorbent assembly 5 onto the inner sheet 62 to form folded-over portions 61a, 61b, 62a, and 62b.

As shown in Fig. 2, the outer cover 6 has its both lateral side edges concavely curved in the longitudinally (Y-directionally) middle part thereof. That is, the outer cover 6 has its longitudinally middle part narrowed. As shown in Figs. 1 to 3, the outer cover 6 is composed of the outer sheet 61 defining the exterior surface of the diaper, the inner sheet 62 overlaid on the inner side of the outer sheet 61, and elastic members fixedly arranged between the two sheets 61 and 62. The elastic members include a plurality of waist elastic members 63, a plurality of leg elastic members 64, and a plurality of below-waist elastic members 65.

The waist elastic members 63 are disposed in the peripheral part of the front section A and the rear section B along the waist opening WO. Each below-waist elastic member 63 is fixed in its extended state in the transverse direction of the diaper 1. The transverse direction of the diaper 1 while worn is the same as the circumferential direction of the waist or below-waist part of a wearer. The transverse direction of the diaper 1 (the direction perpendicular to the centerline CL of the diaper 1 in its flat-out, uncontracted state) will hereinafter be referred to as the X-direction.
The leg elastic members 64 are disposed in the peripheral part of each leg openings LO as shown in Fig. 2. Each leg elastic member 64 is fixed in its extended state along the circumferential direction of the leg opening LO.
The below-waist elastic members 65 are disposed between the waist elastic members 63 and the end of the crotch section C in each of the front section A and the rear section B as shown in Fig. 2. The below-waist elastic members 65 are separately provided on both sides of the centerline CL, each being fixed along the transverse direction of the diaper 1.

As shown in Figs. 2, 3(a), and 3(b), the sheet material forming the outer sheet 61 of the diaper 1 has a length extending in the longitudinal direction (Y direction) outward from the area where the waist elastic members 63 are held between the outer sheet 61 and the inner sheet 62 in each of the front section A and the rear section B. The extended part of the outer sheet 61 is folded back over the peripheral edge We of the waist opening WO onto the inner sheet 62 to provide folded-over portions 61 a and 61b.
As shown in Figs. 2 and 3(a), the folded-over portion 61a extends such that the longitudinal (Y-directional) end 61a1 thereof reaches beyond the longitudinal (Y-directional) end of the absorbent assembly 5 in the front section A toward the crotch section C. Similarly, the folded-over portion 61b extends such that the longitudinal (Y-directional) end 61b1 thereof reaches beyond the longitudinal (Y-directional) end of the absorbent assembly 5 in the rear section B toward the crotch section C as shown in Figs. 2 and 3(b).

As shown in Figs. 2, 3(a), and 3(b), the sheet material forming the inner sheet 62 of the diaper 1 has a length extending in the longitudinal direction (Y direction) outward from the area where the waist elastic members 63 are held between the outer sheet 61 and the inner sheet 62 in each of the front section A and the rear section B. The extended part of the inner sheet 62 is folded back onto itself over the peripheral edge We of the waist opening WO as a unit with the outer sheet 61 to provide the folded-over portions 62a and 62b.
As shown in Figs. 2 and 3(a), the folded-over portion 62a extends such that the longitudinal (Y-directional) end 62al thereof reaches near the midpoint between the peripheral edge We of the waist opening WO and the end of the absorbent assembly 5 in the front section A. Similarly, the folded-over portion 62b extends such that the longitudinal (Y-directional) end 62bl thereof reaches near the midpoint between the peripheral edge We of the waist opening WO and the end of the absorbent assembly 5 in the rear section B as shown in Figs. 2 and 3(b).
That is, the length in the longitudinal direction (Y direction) of the folded-over portions 62a and 62b is shorter than that of the folded-over portions 61a and 61b.

As shown in Fig. 2, one longitudinal (Y-directional) end of the absorbent assembly 5 is fixed between the inner sheet 62 and the folded-over portion 61a of the outer sheet 61 in the transverse middle part each of the front section A and the rear section B. The length in the longitudinal direction (Y-direction) of the folded-over portions 62a and 62b in the front section A and the rear section B, respectively, is the same (substantially the same) over the whole width (in the transverse direction) of the front section A and the rear section B, respectively.

As shown in Fig. 3(a), the lateral side edge portion 6a of the front section A has a region 6a1 formed of a stack of four sheets (i.e., the outer sheet 61, the inner sheet 62, the folded-over portion 61a, and the folded-over portion 62a), a region 6a2 formed of a stack of three sheets (i.e., the outer sheet 61, the inner sheet 62, and the folded-over portion 61a), and a region 6a3 formed of a stack of two sheets (i.e., the outer sheet 61 and the inner sheet 62) along the longitudinal direction (Y-direction). The lateral side edge portion 6b of the front section B has the same structure. That is, as shown in Fig. 3(b), the lateral side edge portion 6b has a region 6b1 formed of a stack of four sheets (i.e., the outer sheet 61, the inner sheet 62, the folded-over portion 61b, and the folded-over portion 62b), a region 6b2 formed of a stack of three sheets (i.e., the outer sheet 61, the inner sheet 62, and the folded-over portion 61b), and a region 6b3 formed of a stack of two sheets (i.e., the outer sheet 61 and the inner sheet 62) along the longitudinal direction (Y-direction).

On overlapping the side edge portion 6a and the side edge portion 6b, the resultant overlapped portion 7 includes a region 71 formed by overlapping the regions 6a1 and 6b1 and having a stack of eight sheets, a region 72 formed by overlapping the regions 6a2 and 6b2 and having a stack of six sheets, and a region 73 formed by overlapping the regions 6a3 and 6b3 and having a stack of four sheets. In the diaper 1, the region 71 is provided along the periphery of the waist opening WO as a waist end region 71, in which a larger number of sheets are stacked than in the other regions 72 and 73 of the overlapped portion 7.

In the diaper 1, the stacked sheet structure of the waist end region 71 of the overlapped portion 7 in the front section A and in the rear section B extending over the distance 71a between the opposing two overlapped portions 7 of the front section A and over the distance 71b between the opposing two overlapped portions 7 of the rear section B as shown in Fig 2. More specifically, the lateral side edge portion 6a of the front section A that forms the waist end region 71 of the overlapped portion 7 has a four-layer structure composed of the outer sheet 61, the inner sheet 62, the folded-over portion 61 a, and the folded-over portion 62a (the region 6a1 of Fig. 3(a)) as stated above. The four-layer structures described is common to the lateral side edge portions 6a and 6a on either side of the centerline CL. In addition to this, the same stacked sheet structure as with the lateral side edge portions 6a and 6a is continuously present between the lateral side edge portions 6a and 6a of the front section A where no overlapped portion 7 is formed.
The rear section B forming the waist end region 71 of the overlapped portion 7 has the same stacked sheet structure as discussed above. As described, the lateral side edge portion 6b of the rear section B has a four-layer structure composed of the outer sheet 61, the inner sheet 62, the folded-over portion 61b, and the folded-over portion 62b (the region 6b1 of Fig. 3(b)). This is common to the lateral side edge portions 6b and 6b on either side of the centerline CL. In addition to this, the same stacked sheet structure as with the side edge portions 6b and 6b is continuously present between the side edge portions 6b and 6b of the rear section B where no overlapped portion 7 is formed.

The expression "the same stacked sheet structure" does not take the overlapped state of the sheets into consideration. It suffices that the sheets making up the front section A in the overlapped portions or the sheets making up the rear section B in the overlapped portions extend laterally inward in the front section A or the rear section B and that the number of the sheets stacked in that extended area in the front section A or the rear section B is the same as the number of the sheets constituting the overlapped portions.

A part of the front section A or the rear section B in the transverse direction (X-direction) may have a different stacked sheet structure from the rest, but it is preferred that at least 50%, more preferably 70% or more, of the transverse distance between the opposing overlapped portions 7 of the front section A and the rear section B (taken as 100%) have the same stacked sheet structure as the overlapped portions 7. It is preferred that at least the portion between the overlapped portion 7 and the adjacent lateral side edge (inclusive of an extension from the lateral side edge) of the absorbent assembly in the transverse direction (X-direction) in each of the front section A and the rear section B have the same stacked sheet structure as the respective lateral side edge portion.
Back to the diaper 1 of the present embodiment, the same stacked sheet structures as every region 71, 72, and 73 in the longitudinal direction (Y-direction) of the overlapped portion 7 continue over the whole width of the front section A and the rear section B.

The waist end region 71 having more sheets than the other regions 72 and 73 preferably has a length L1 of 3 to 30 mm, more preferably 5 to 25 mm, even more preferably 10 to 20 mm, in the longitudinal direction of the side seal 8 (Y-direction), so as to provide the peripheral portion of the waist opening WO with good cushioning or feel to the touch.

It is preferred that the sheet material forming the outer sheet 61 and the sheet material forming the inner sheet 62 be folded over as a stacked unit along the peripheral edge We of the waist opening as with the case of the diaper 1 of the present embodiment. This is preferred to provide the peripheral portion of the waist opening WO with good cushioning or feel to the touch. The way of folding described also offers an advantage that, where needed, a waist elastic member 63 may easily be disposed at almost the same position at the peripheral edge We of the waist opening WO.
In order to secure good texture and cushioning of the waist end region, it is desirable to minimize use of an adhesive in that region. The amount of the adhesive used in that region is preferably in the range of from 0 to 10 g/m². Methods for avoiding use of an adhesive include fusion bonding the outer sheet and the inner sheet by, for example, heat sealing or ultrasonic sealing in parts where elastic members are absent.

The side seals 8 of the diaper 1 are formed by fusion bonding the overlapped portions 7.
As shown in Fig. 3(c), the side seal 8 has a seal region 8a1 formed by fusion bonding the waist end region 71 having a 8-sheet stack, a seal region 8a2 formed by fusion bonding the 6-sheet stack region 72, and a seal region 8a3 formed by fusion bonding the 4-sheet stack region 73. A plurality of fusion bonds 81 each extending in the transverse direction (X-direction) are formed in each of the seal regions 8a1, 8a2, and 8a3.
As shown in Fig. 3(c), the fusion bonds 81 in the seal region 8a1 each have the same width (d) as the fusion bonds 81 in the seal regions 8a2 and 8a3. However, the pitch P of the fusion bonds 81 in the seal region 8a1 is larger than the pitch P' of the fusion bonds 81 in the seal regions 8a2 and 8a3.

Since the diaper 1 of the present embodiment has the fusion bonds 81 in the above described pattern, the seal region 8a1 has a smaller total length D1 of the fusion bonds 81 per unit length in its longitudinal direction as measured along the skin side edge 8e of the side seal 8 than the equivalent total lengths D2 and D3 of the other seal regions 8a2 and 8a3, respectively. That is, D1<D2, and D1<D3.

As used herein, the term "skin side edge 8e" of the side seal is one of the side edges in the transverse direction of the side seal 8 (the same as the transverse direction of the diaper 1 or the X-direction) that is closer to the wearer's skin while the diaper is worn. More concretely, the skin side edge 8e is one of the two side edges in the X-direction of the side seal 8 that is closer to the centerline CL.

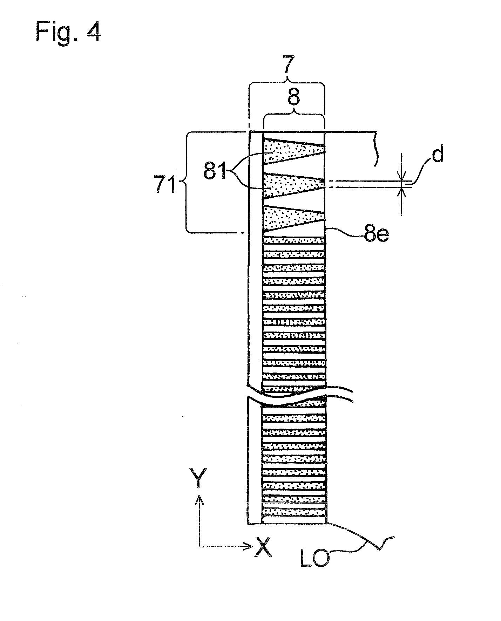

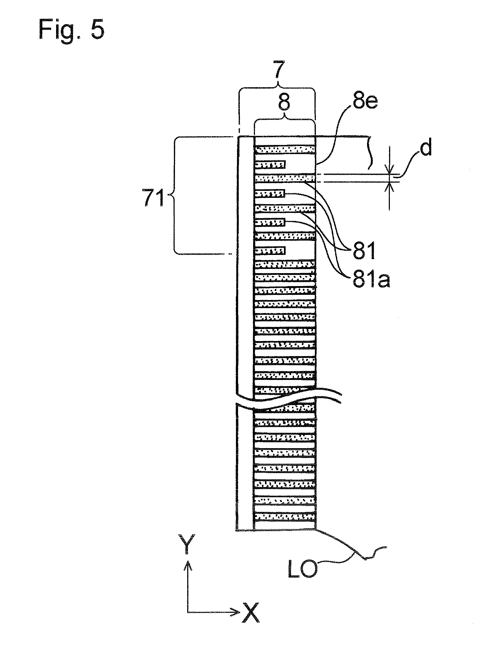

When the length of a fusion bond 81 in the longitudinal direction (Y-direction) of a side seal 8 is different between the opposite side edges in the transverse direction (X-direction) of the side seal 8 as with the case shown in Fig. 4, the length d in the longitudinal direction (X-direction) along the edge closer to the wearer's skin (the right-hand side in Fig. 4) is used to calculate the total lengths D1 to D3.
When a side seal 8 has fusion bonds 81 reaching the skin side edge 8e of the side seal 8 and fusion bonds 81 a short of reaching the skin side edge 8e of the side seal 8 as with the case shown in Fig. 5, the fusion bonds 81a are regarded as having no length along the skin side edge 8e of the side seal 8, and the total lengths D1 to D3 are calculated only from the length d of the fusion bonds 81 reaching the skin side edge 8e.

The above-defined total length D1 (i.e., the total length per unit length in the longitudinal direction of the side seal (Y-direction)) of the fusion bonds 81 in the seal region 8a1 (the seal region formed by fusion bonding the waist end region 71) along the skin side edge 8e of the side seal is obtained as follows. The length d of the individual fusion bonds 81 formed in a region from the peripheral edge We of the waist opening WO to a position 10 mm below the peripheral edge We is measured along the longitudinal direction (Y-direction). The total of the lengths d of these fusion bonds 81 is divided by 10 mm to give D1. When the distance L1 from the peripheral edge We of the waist opening WO to the lower end of the waist end region 71 is shorter than 10 mm, the total length D1 is obtained by adding up the lengths d of the fusion bonds 81 present in the distance L1 from the peripheral edge We of the waist opening WO measured along the longitudinal direction (Y-direction) and dividing the sum by the distance L1 to give D1.

The total lengths D2 and D3 (i.e., the total length per unit length in the longitudinal direction of the side seal (Y-direction)) of the fusion bonds 81 in the seal regions 8a2 and 8a3 (the seal regions formed by fusion bonding the regions 72 and 73 below the waist end region 71), respectively, along the skin side edge 8e of the side seal are obtained by measuring the length d of the individual fusion bonds 81 present in 10 mm-long regions below the waist end region 71 and dividing the total of the lengths d in each region by 10 mm. A 10 mm length of the 10 mm-long region is preferably from a midpoint between two fusion bonds 18 adjacent in the longitudinal direction (Y-direction) in each of the seal region 8a2 and 8a3 to 10 mm down (toward the leg opening).

While in the diaper 1 the seal regions 8a2 and 8a3 have the same arrangement of fusion bonds 81 so that the total length D2 in the seal region 8a2 and the total length D3 in the seal region 8a3 are equal, the total length D2 in the seal region 8a2 may be larger than the total length D3 of the seal region 8a3, or vice versa. It is preferred, nevertheless, that the total length D1 in the seal region 8a1 be smaller than at least the total length D2 in the seal region 8a2 that is contiguously below the seal region 8a1, and it is more preferred that the total length D1 be smaller than either of the total lengths D2 and D3.

In the present embodiment, the length d of the individual fusion bonds 81 in the seal region 8a1 and the seal regions below the seal region Sa1 (i.e., the seal regions 8a2 and 8a3) is preferably 0.2 to 3 mm, more preferably 0.5 to 2.5 mm. The ratio of the length d of the fusion bonds in the seal region 8a1 to the length d of the fusion bonds in the seal regions 8a2 and 8a3 below the seal region 8a1 (the former d/the latter d) is preferably 0.5 to 1.5, more preferably 0.7 to 1.2.
The fusion bonds 81 in the seal region 8a1 preferably have a pitch P (the distance between the center of a fusion bond and the center of an adjacent fusion bond in the longitudinal direction) of 0.5 to 4.0 mm, more preferably 0.7 to 3.0 mm. The fusion bonds 81 in the seal regions 8a2 and 8a3 preferably have a pitch P' of 1 to 5 mm, more preferably 1.5 to 4 mm. The ratio of the pitch P in the seal region 8a1 to the pitch P' in the seal regions 8a2 and 8a3 below the seal region 8a1 (P/P') is preferably 1 to 4, more preferably 2.0 to 3.5.

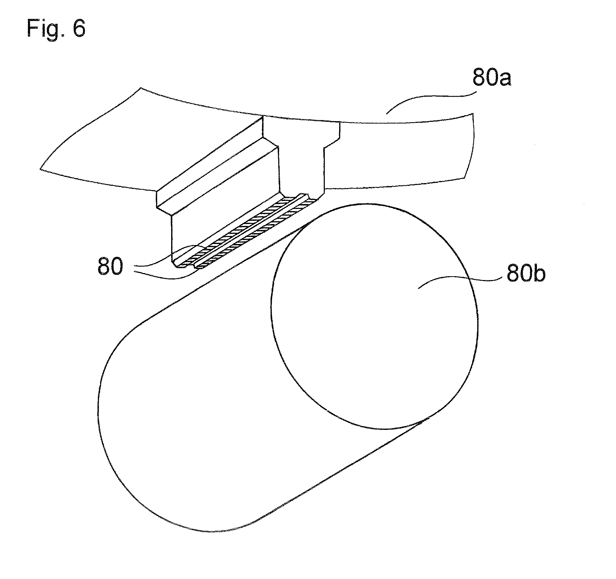

Methods for fusion bonding to form the side seal 8 include heat embossing, ultrasonic embossing, and high frequency embossing. In the production of the diaper 1, side seal 8 is formed by using a pair of ultrasonic embossing rollers 80a and 80b shown in Fig. 6.
Specifically, a continuous form of the diaper 1 is passed through the nip between an embossing roller 80a having projections 80 shaped to the pattern of the side seal 8 shown in Fig. 3(c) and a flat roller 80b having a flat surface, whereby the overlapped portions 7 formed by overlapping the lateral side edge portion 6a and the lateral side edge portion 6b are embossed between the projections 80 and the flat roller 80b to form the side seals 8. Each projection 80 has small projections corresponding to the fusion bonds 81 of the seal regions 8a1, 8a2, and 8a3 along the direction perpendicular to the moving direction of the continuous form of the diaper 1, the direction perpendicular to the moving direction coinciding with the longitudinal direction of the side seal 8 (Y-direction) shown in Fig. 3(c). Thus, one side seal 8 is formed by a single pass through the embossing nip.

The fusion bonds 81 in the side seal 8 are depressions formed by the small projections on the projection 80, where the facing sides of the lateral side edge portion of the front section A and the lateral side edge portion of the rear section B are fused together. The fusion bonds 81 are usually cohesive film-like parts resulting from embossing and are the deepest parts from the surface of the front section A or the rear section B. The "width" of the side seal 8 is the largest of the dimensions of the fusion bonds in the X-direction. When the side seal 8 is contracted in the transverse direction (X-direction) by the below-waist elastic member 63 and the like, the length d of the fusion bond 8 in the longitudinal direction is measured with the side seal 8 stretched flat.

It is preferred that the fusion bond strength of the side seal 8 in the waist end region 71 be lower than that in the regions 72 and 73 below the waist end region 71.
The fusion bond strength in the waist end region 71 is the fusion bond strength of the seal region 8a1. It is preferred that the seal region 8a1 have a lower fusion bond strength than either of the seal region 8a2 and the seal region 8a3.

The higher the fusion bond strength of the side seal 8 is, the stiffer the side seal 8 tends to feel to the wearer's skin.
While a diaper is worn, because the wearer's waist circumference largely varies during wear, the waist end region of the diaper is more liable than the other regions to give a wearer an uncomfortable feeling. When the fusion bond strength in the waist end region 71 is lower than that of the regions 72 and/or 73 below the region 71, the discomfort caused by the waist end region is eliminated.

From this viewpoint, the fusion bond strength in the waist end region 71 is preferably 0.3 to 3 N/5 mm, more preferably 0.3 to 4.0 N/5 mm, and that of the region 72 or 73 below the waist end region 71 is preferably 2 to 6 N/5 mm, more preferably 2.5 to 5.0 N/5 mm. The ratio of the former to the latter (the former/the latter ) is preferably 0.2 to 0.9, more preferably 0.3 to 0.6.

### Method for measuring fusion bond strength:

A side seal 8 is cut out of a diaper 1 along the longitudinal direction of the diaper 1 (Y-direction). The cutting line is about 30 mm inward (toward the centerline CL) from the side seal 8 in both the front section A and the rear section B.
The cut out side seal 8 is cut along the transverse direction (X-direction) to make a specimen measuring 5 mm in the longitudinal direction (Y-direction) from each of the seal regions 8a1, 8a2, and 8a3.
The seal strength of the specimen from each seal region is measured using a tensile tester (Autograph AGS50A, from Shimadzu Corp.). The measurement is carried out by gripping the free ends of the front section and the rear section of the specimen, i.e., the portions closer to the centerline CL than the side seal 8, pulling the jaws in opposite directions (T peel) at a pulling speed (rate of jaw separation) of 300 mm/min, and reading the maximum load observed. The measurement is taken on five specimens (n=5) to obtain an average, which is taken as the fusion bond strength of that region. If a region is too short to make a 5-mm wide specimen, the measurement is taken on a specimen cut out with a width as close to 5 mm as possible, and the reading is converted to a fusion bond strength per 5 mm width.
The direction of peeling the side seal 8 of the specimen is the same as the transverse direction of the side seal 8 (X-direction). When the peel strength varies in the peel direction as with the cases shown in Figs. 4 and 5, the measurement starts at the time when the peel strength reaches 0.1 N and ends when the jaws separate from each other by a distance of 5 mm from the starting point, and the maximum load observed during the measurement is taken as the fusion bond strength of the specimen.

As shown in Fig. 2, the absorbent assembly 5 is substantially oblong and includes a liquid permeable topsheet 2, a liquid impermeable or water repellent backsheet 3, and a liquid retentive absorbent member 4 interposed between the topsheet 2 and the backsheet 3. As shown in Fig 2, the absorbent assembly 5 is disposed to straddle the rear section B and the front section A of the outer cover 6. Both longitudinal (Y directional) ends of the absorbent assembly 5 are positioned longitudinally (Y directionally) inward from the respective longitudinal (Y directional) ends of the outer cover 6. The absorbent assembly 5 is overlapped with an adhesive, heat sealed, ultrasonic sealed or otherwise attached to the inner sheet 62 of the outer cover 6.

As shown in Fig. 2, the absorbent assembly 5 is provided with a pair of side cuffs 51 and 51, each formed of a liquid resistant or water repellent and breathable material, along both longitudinally (Y directionally) extending sides thereof. Each side cuff 51 has, along near its free end, a side cuff-forming elastic member 52 fixed in its stretched state, so that, while the diaper is worn, the side cuffs 51 will stand with their free end up to prevent excrement from running off in the transverse direction (X-direction) of the absorbent assembly 5. The sheet material forming the side cuff 51 has a lateral extension of a prescribed width (in the direction x) from the absorbent assembly 5 wrapped around onto the garment facing side of the absorbent assembly 5 and secured there.

The above described diaper 1 can be worn in the same way as with conventional disposable pull-on diapers.
As stated previously, the diaper 1 has, in the waist opening WO-side end, the waist end region 71 having a larger number of sheets than the other regions 72 and 73, and the sheet stacked structure of the waist end region 71 in each of the front section A and the rear section B extends over the distance 71a between opposing overlapped portions of the front section A and over the distance 71b between opposing overlapped portions of the rear section B. As a result, the portion of the diaper 1 that is to come into contact with the wearer's whole waist circumference will give the wearer good cushioning or feel to the touch.
While a wearer especially an infant or a child wears a diaper, the part of the wearer's body to which the waist end region of the diaper applies has a larger circumferential length than the other body parts to which the diaper applies. Moreover, that part of the body considerably changes in circumference with the movement of standing or sitting and before and after a meal. Therefore, that part of the body is the most influenced by the softness of the diaper per se, and it is very important for the corresponding waist end region of the diaper to be soft. In addition to this, the waist end region of a diaper is a portion where a diaperer, e.g., a baby's mother put her hands to widen the waist opening in putting the diaper on a wearer. Therefore, it is advantageous for the waist end region to be soft in terms of not only comfort to a wearer but also improved touch to the diaperer's skin.

On the other hand, as the number of the members forming the overlapped portion 7 increases, it generally follows that the amount of a fused resin increases. On firmly fusion bonding such a overlapped portion 7, the increased amount of heat fused resin renders that portion harder, and the hardened portion can come into contact with a waist, a hand, a leg, and other body parts to cause irritation when a diaperer widens the waist opening in putting the diaper on a wearer or while the diaper is worn.
In the diaper 1, the total length D1 of the fusion bonds 81 per unit length in the longitudinal direction in the seal region 71 formed by fusion bonding the waist end region as measured along the skin side edge 8e of the side seal is smaller than either of the equivalent total lengths D2 and D3 in the seal regions formed by fusion bonding the regions 72 and 73 below the waist end region 71. As a result, the hardened regions of the side seal 8 are prevented from coming into contact with a wearer's skin, and yet a certain fusion bond strength is secured in the waist end region 71. The side seal is thus prevented from giving a wearer discomfort or adversely affecting the feel to the wearer's and/or diaperer's skin during diapering or wear.

A disposable pull-on diaper 101 incorporating a second embodiment of the invention (hereinafter also referred to as a diaper 101) will then be described. The description will generally be confined to the differences from the diaper 1 of the first embodiment. The same elements or members are identified with the same reference numerals and will not be described redundantly. The description of the diaper 1 applies as appropriate to the undescribed details of the diaper 101.

Similarly to the diaper 1, the diaper 101 of the second embodiment has a pair of side seals 108 and 108, which are formed by overlapping both lateral side edge portions 6a and 6a of the front section A and both lateral side edge portions 6b and 6b of the rear section B and fusion bonding the overlapped portions 7 and 7.

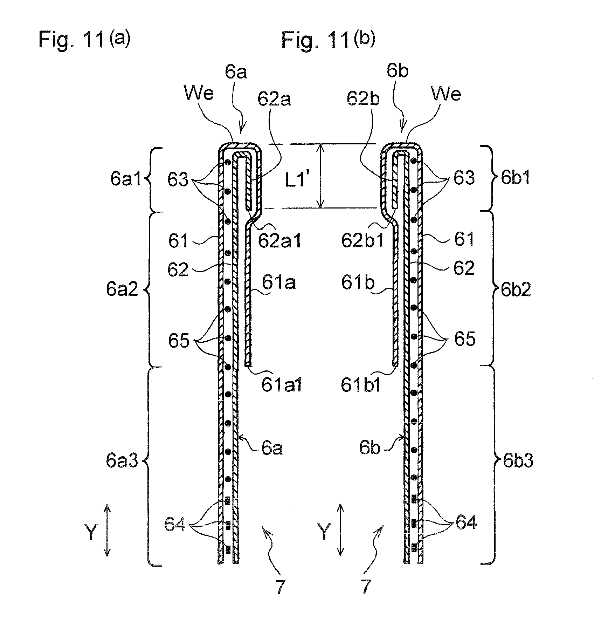

As shown in Fig. 11(a), the lateral side edge portion 6a of the front section A has a region 6a1 formed of a stack of four sheets (i.e., an outer sheet 61, an inner sheet 62, a folded-over portion 61a, and a folded-over portion 62a), a region 6a2 formed of a stack of three sheets (i.e., the outer sheet 61, the inner sheet 62, and the folded-over portion 61a), and a region 6a3 formed of a stack of two sheets (i.e., the outer sheet 61 and the inner sheet 62) along the longitudinal direction (Y-direction). The lateral side edge portion 6b of the front section B has the same structure. That is, as shown in Fig. 11(b), the lateral side edge portion 6b has a region 6b1 formed of a stack of four sheets (i.e., the outer sheet 61, the inner sheet 62, the folded-over portion 61b, and the folded-over portion 62b), a region 6b2 formed of a stack of three sheets (i.e., the outer sheet 61, the inner sheet 62, and the folded-over portion 61b), and a region 6b3 formed of a stack of two sheets (i.e., the outer sheet 61 and the inner sheet 62) along the longitudinal direction (Y-direction).

The overlapped portion 7 in the diaper 101 has a region 71 formed of a stack of eight sheets (an overlapped portion of the regions 6a1 and 6b1), a region 72 formed of a stack of six sheets (an overlapped portion of the regions 6a2 and 6b2), and a region 73 formed of a stack of four sheets (an overlapped portion of the regions 6a3 and 6b3).
The side seal 108 of the diaper 101 is formed by fusion bonding the overlapped portion 7 having the above described structure.

In making the diaper 101 of the second embodiment, fusion bonding the overlapped portion 7 is carried out by pressing the overlapped portion 7 between two sealing rollers each having projections.
The side seal 108 of the diaper 101 has a front side seal pattern 8a shown in Fig. 12(a) on the side of the front section A and a rear side seal pattern 8b shown in Fig. 12 (c) on the side of the rear section B. As is apparent from Figs. 12(a) and 12(c), the front side seal pattern 8a and the rear side seal pattern 8b are different from each other.

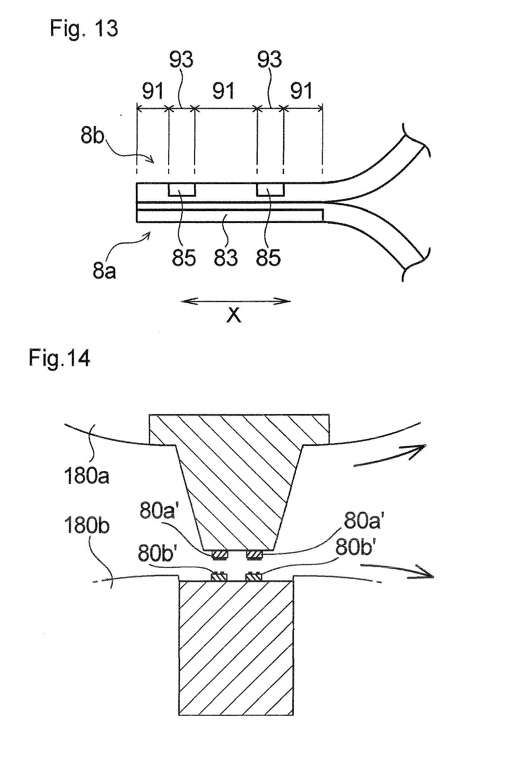

As shown in Figs. 12(a) and 13, the front side seal pattern 8a is composed of a number of wider-than-long depressions 83 and 84 each extending in the transverse direction of the side seal 108 (X-direction). The many wider-than-long depressions 83 and 84 are spacedly arrayed in the longitudinal direction of the side seal 108 (Y-direction).
As shown in Figs. 12(c) and 13, on the other hand, the rear side seal pattern 8b is composed of two longer-than-wide depressions 85 each extending in the longitudinal direction of the side seal 108 (Y-direction). The two longer-than-wide depressions 85 are spaced in the transverse direction (X-direction), and each of them is spaced from the lateral edge of the side seal 108, particularly from the skin side edge 8e.
As used herein, the term "skin side edge 8e" of the side seal is one of the lateral side edges in the transverse direction of the side seal 8 (the same as the transverse direction of the diaper 101 or the X-direction) that is closer to the wearer's skin while the diaper is worn. More concretely, the skin side edge 8e is one of the opposite side edges in the X-direction of the side seal 108 that is closer to the centerline CL extending in the longitudinal direction of the diaper (i.e., the direction from the front section A through the crotch section C to the rear section B).

As shown in Fig. 12(a), the side seal 108 of the diaper 101 includes an end region 181 that is nearer to the waist opening and the other region 182 below the end region 181, and the front side seal pattern 8a in the end region 181 and that in the other region 182 are different from each other. The expression "the seal pattern in the end region 181 and that in the other region 182 are different from each other" includes not only the case in which the pitch or the dimension of the wider-than-long depressions 83 and 84 in the longitudinal direction in the end region 181 are different from that in the other region 182 but also the case in which no depressions are formed in the end region 181 by pressure application from the front side A while the other region 182 has wider-than-long depressions 84.

On the other hand, the rear side seal pattern 8b is the same between the end region 181 in the waist opening WO side and the other region 182 of the side seal 108 as is seen from Fig. 12(c). More concretely, the two longer-than-wide depressions 85 continuously extend, straddling the end region 181 and the other region 182.

Methods for fusion bonding to form the side seal 108 include heat embossing, ultrasonic embossing, and high frequency embossing. In making the diaper 101, side seal 108 is formed by using a pair of ultrasonic embossing rollers (sealing rollers) 180a and 180b shown in Fig. 14.
As shown in Fig. 14, the embossing roller 180a has a block on its peripheral surface, and the block has on its top surface two projections 80a' each extending in the axial direction of the roller. Each projection 80a' has on its top surface a row of a number of small projections 83' and 84' corresponding to the wider-than-long depressions 83 and 84 described above (see Fig. 12(b)) in a pattern corresponding to the front side seal pattern 8a shown in Fig. 12(a). The individual small projections 83' and 84' extend in the peripheral direction of the roller. The axial direction of the embossing roller 180a and the embossing roller 180b coincide with the longitudinal direction of the side seal 108 (the vertical direction in Figs. 12(a), 12(c), and 12(d)).

The embossing roller 180b has a block on its peripheral surface, and the block has on its top surface two projections 80b', each extending in the axial direction of the roller. Each projection 80b' has on its top surface two small ridge-shaped projections 85' corresponding to the longer-than-wide depressions 85 described above (see Fig. 12(d)) in a pattern corresponding to the rear side seal pattern 8b shown in Fig. 12(c). The small projections 85' are spaced apart from each other in the peripheral direction of the roller.
The two projections 80a' of the embossing roller 180a and the two projections 80b' of the embossing roller 180b are designed to simultaneously embossing adjacent lateral side edge portions of two adjacent diapers in a continuous form of the diaper 1. After the adjacent lateral side edge portions are embossed to form side seals, the continuous form of the diaper assembly is cut between the two side edge portions to provide individual diapers.

On pressing the overlapped portion 7 of the front side edge portion 6a and the rear side edge portion 6b between the embossing rollers 180a and 180b, the regions pressed by the small projections 83' and 84' of the embossing roller 180a become the wider-than-long depressions 83 and 84, respectively, and, at the same time, provide weakly bonded regions 91 where the side edge portions 6a and 6b are weakly bonded by pressure application substantially only from the side of the front section A (see Figs. 12(e) and 13).
Similarly, the regions pressed by the small projections 85' of the embossing roller 180b become the longer-than-wide depressions 85 and, at the same time, provide weakly bonded regions 92 where the side edge portions 6a and 6b are weakly bonded by pressure application substantially only from the side of the rear section B (see Figs. 12(e) and 13).
On the other hand, the regions pressed from both the side of the front section A and the side of the rear section B by the small projections 83' or 84' of the embossing roller 180a and the small projections 85' of the embossing roller 180b become strongly bonded regions 93 where the side edge portions 6a and 6b are firmly bonded, In the strongly bonded regions 93, the resin component of the constituent sheets usually has melted and solidified to become cohesive and film-like as a result of embossing. It is preferred that, in the weakly bonded regions 91 and 92, the constituent fibers are weakly fusion bonded together, entangled with one another, or otherwise overlapped weakly.
The regions pressed from both the side of the front section A and the side of the rear section B (i.e., the strongly bonded regions 93) are interpreted as constituting part of the front side seal pattern and part of the front side seal pattern. The sheets in other regions of the overlapped portion 7 having been pressed by none of the small projections 83 to 85 are not substantially overlapped or are weakly overlapped by the presence of the weakly bonded regions.

Since the side seal 108 of the diaper 101 of the second embodiment has different seal patterns between the front side pattern 8a and the rear side pattern 8b, the strongly bonded regions 93 are formed at the overlaps of the two patterns and therefore distributed in the planar direction of the side seal 108. Such an arrangement of the strongly bonded regions 93 prevents the side seal 108 from becoming hard as a whole while securing the bonding strength by the strongly bonded regions 93 so that the side seal 108 may not be unintentionally torn apart.
Additionally, since the side seal 108 of the diaper 101 of the second embodiment has different seal patterns within the front side seal pattern 8a between the waist opening side end region 181 and the other region 182, the fusion bonds, particularly the strongly bonded regions 93 of the side seals are effectively prevented from giving a wearer discomfort or irritating the skin of a wearer or a diaperer even when a downward or sideways (in a direction vertical to the wearer's skin) pressing force is exerted to the waist opening peripheral portion in putting on the diaper 101 or putting the diaper 101 on or during wearing the diaper 101.

In the diaper 101 of the second embodiment, the pitch P11 of the wider-than-long depressions 83 in the waist opening WO-side end region 181 is larger than the pitch P12 of the wider-than-long depressions 84 in the other region 182. As a result, the fusion bond strength of the end region 181 is smaller than that of the other region 182.

The higher the fusion bond strength of the side seal 108 is, the stiffer the side seal 108 tends to feel to the wearer's skin.
Since the end region 181, which is located along the edge of the waist opening WO, has a lower fusion bond strength than the region 182 below the end region 181, the waist opening-side end portion is prevented more effectively from giving a wearer discomfort or irritating the skin of a wearer or a diaperer in putting on the diaper 101 or putting the diaper 101 on or during wearing the diaper 101.

The waist opening-side end region 181 preferably has a fusion bond strength of 0.3 to 3 N/5 mm, more preferably 0.3 to 4.0 N/5 mm, and the other region 182 below the end region 181 preferably has a fusion bond strength of 2 to 6 N/5 mm, more preferably 2.5 to 5.0 N/5 mm. The ratio of the fusion bond strength of the end region 181 to that of the other region 182 (the former/the latter) is preferably 0.2 to 0.9, more preferably 0.3 to 0.6.

### Method for measuring fusion bond strength:

A side seal 108 is cut out of a diaper 101 along the longitudinal direction of the diaper 101 (Y-direction). The cutting line is about 30 mm inward (toward the centerline CL) from the side seal 108 in both the front section A and the rear section B.
The cut out side seal 108 is cut along the transverse direction (X-direction) to make a specimen measuring 5 mm in the longitudinal direction (Y-direction) from each of the end region 181 and the other region 182.
The seal strength of the specimen from each region is measured using a tensile tester (Autograph AGS50A, from Shimadz Corp.). The measurement is carried out by gripping the free ends of the front section and the rear section of the specimen, i.e., the portions closer to the centerline CL than the side seal 108, pulling the jaws in opposite directions (T peel) at a pulling speed (rate of jaw separation) of 300 mm/min, and reading the maximum load observed. The measurement is taken on five specimens (n=5) to obtain an average, which is taken as the fusion bond strength of that region. If a region is too short to make a 5-mm wide specimen, the measurement is taken on a specimen cut out with a width as close to 5 mm as possible, and the reading is converted to a fusion bond strength per 5 mm width. The direction of peeling the side seal 108 of the specimen is the same as the transverse direction of the side seal 108 (X-direction).

The side seal 108 of the diaper 101 has no overlap between the front side pattern 8a and the rear side pattern 8b along its skin side edge 8e. That is, the strongly bonded region 93 described above is not present along the skin side edge 8e. Therefore, the strongly bonded hard regions 93 of the side seal 108 are effectively prevented from coming into contact with the skin of a wearer. The side seals are thus prevented more effectively from giving a wearer discomfort and from adversely affecting the skin of a wearer or a diaperer during diapering or wear.

In the diaper 101, the wider-than-long depressions 83 in the end region 181 of the front side seal pattern 8a have the same length d1 in the longitudinal direction of the side seal 8 (X-direction) and are arranged at a given pitch P11 in that longitudinal direction (Y-direction). Similarly, the wider-than-long depressions 84 in the other region 182 have the same length d1 in the longitudinal direction of the side seal 8 (X-direction) and are arranged at a given pitch P12 in that longitudinal direction (Y-direction).

The length d' of the wider-than-long depressions 83 and 84 is preferably 0.3 to 3.5 mm, more preferably 0.5 to 3.0 mm. The ratio of the length d' of the wider-than-long depressions 83 to the length d' of the wider-than-long depressions 84 (the former/the latter) is preferably 0.2 to 0.8, more preferably 0.3 to 0.5.
The pitch P11 of the wider-than-long depressions 83 is preferably 0.5 to 3.0 mm, more preferably 1.0 to 2.5 mm, and the pitch P12 of the wider-than-long depressions 84 is preferably 1.0 to 5.0 mm, more preferably 2.0 to 4.0 mm. The ratio of the pitch 11 to the pitch 12 (P11/P12) is preferably 0.1 to 3.0, more preferably 0.5 to 2.0.
Each of the longer-than-wide depressions 85 of the front side seal pattern 8b is preferably away from the closer one of the lateral side edges of the side seal (the same as the opposite ends of the wider-than-long depressions 83 and 84 in the X-direction) by at least 1.0 mm, more preferably 2.0 to 5.0 mm.

In order to form a soft region in the waist opening WO-side end portion of the side seal 108 and thereby to prevent the strongly bonded regions from giving a wearer discomfort or irritating the skin of a wearer or a diaperer even when a downward or sideways (in a direction vertical to the wearer's skin) pressing force is exerted to the waist opening peripheral portion, it is preferred for the region of the front side seal pattern 8a different in seal pattern from the other region 182 (i.e., the region located in the end region 181) to have a length L11 of 3 to 30 mm, more preferably 5 to 25 mm, even more preferably 10 to 20 mm, in the longitudinal direction of the side seal 108 (Y-direction).

The region of the front side seal pattern 8a different in seal pattern from the other region 182 (i.e., the region located in the end region 181) is formed in the region 71 where the region 6a1 and the region 6b1 are overlapped to make a stack of the largest number of sheets.
In general, as the number of the members forming the overlapped portion 7 increases, the amount of a fused resin increases. On firmly fusion bonding such a overlapped portion 7, the increased amount of heat fused resin renders that portion harder.
In the diaper 101 of the present embodiment , since the part of the front side seal pattern 8a of the side seal 8 formed in the region having the largest number of sheets is different from the pattern of the lower region, the waist opening peripheral portion having an increased number of sheets is effectively prevented from causing discomfort or irritation to the skin while retaining good cushioning and feel to the touch.

The region 71 with the largest number of sheets has a length L1' spanning from the waist opening WO peripheral edge We to its lower end (the position of the ends 62a1 and 62b1) of 3 to 30 mm, more preferably 5 to 25 mm, even more preferably 10 to 20 mm.
While a wearer especially an infant or a child wears a diaper, the part of the wearer's body to which the waist end region of the diaper is applied has a larger circumferential length than the other body parts to which the diaper is applied. Moreover, that part of the body considerably changes in circumference with the movement of standing or sitting and before and after a meal. Therefore, that part of the body is the most influenced by the softness of the diaper per se, and it is very important for the corresponding waist end region of the diaper to be soft. In addition to this, the waist end region of a diaper is a portion inside of which a diaperer, e.g., a baby's mother put her hands to widen the waist opening in putting the diaper on a wearer. Therefore, it is advantageous for the waist end region to be soft in terms of not only wearer comfort but also improved touch to the diaperer's skin.
In order to secure good texture and cushioning of the end region, it is desirable to minimize use of an adhesive in that region. The amount of the adhesive used in that region is preferably in the range of from 0 to 10 g/m². Methods for avoiding use of an adhesive include fusion bonding the outer sheet and the inner sheet in parts by, for example, heat sealing or ultrasonic sealing in parts where elastic members are absent.

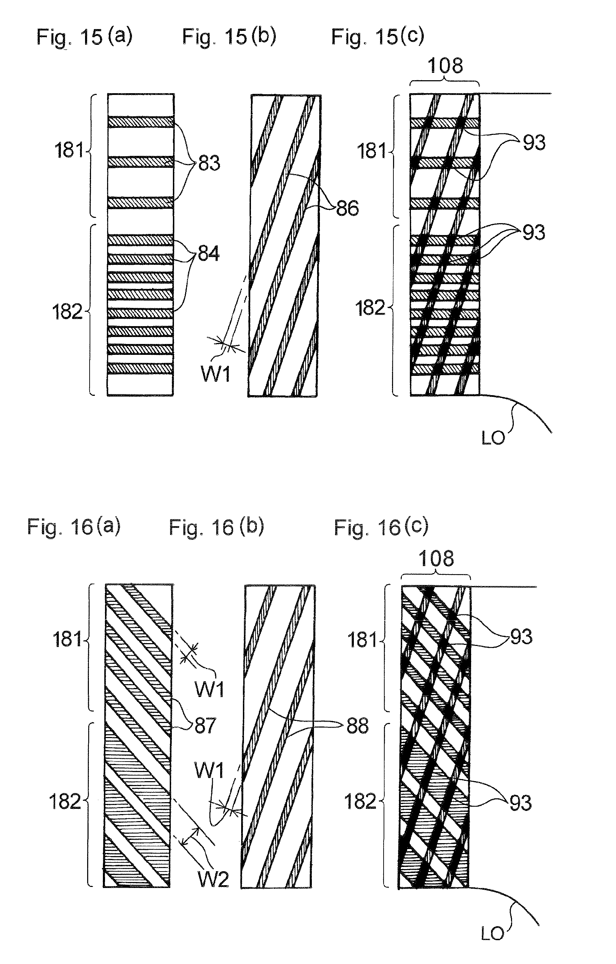

The waist opening-side end region 181 of the side seal 108 in the diaper 101 of the second embodiment corresponds to the seal region 8a1 of the diaper 1 of the first embodiment which is formed by fusion bonding the waist end region 71. The other region 182 below the end region 181 of the side seal 108 in the second embodiment corresponds to the seal regions 8a2 and 8a3 of the side seal 8 in the first embodiment. The side seal 8 of the diaper 1 according to the first embodiment may be designed similarly to the side seal 108 of the diaper 101 according to the second embodiment or the side seal 108 shown in Figs. 15(c) and 16(c) hereinafter described. That is, the side seal 8 may have different patterns between the front side seal pattern and the rear side seal pattern, and the front side seal pattern and/or the rear side seal pattern may have different seal patterns between the seal region 8a1 (corresponding to the end region 181) and the other seal regions 8a2 and 8a3 (corresponding to the region 182). The side seal 8 of the diaper 1 of the first embodiment may also be designed to have a lower fusion bond strength in the seal region 8a1 than the other portions 8a2 and 8a3 similarly to the side seal 108 of the diaper 101 of the second embodiment. The other structures of the overlapped portion 7 and the side seal 108 of the diaper 101 of the second embodiment may be added to the overlapped portion 7 and the side seal 8 of the diaper 1 of the second embodiment.

The materials making up the diapers 1 and 108 will be described.
The sheet materials making up the overlapped portion 7 economically preferably contain a widely employed resin, such as polypropylene, polyethylene, or polyester, as a fusible component. The fusible components of the sheets making up the overlapped portion 7 are preferably of the same type or base. The members constituting the overlapped portion 7 of the diaper 1 or 108 are the outer sheet 61 (including the folded-over portion 61a) and the inner sheet 62 (including the folded-over portion 62a). That is, the fusible component of the outer sheet 61 and that of the inner sheet 62 are preferably of the same base. When the fusible components of the outer sheet 61 and the inner sheet 62 are polypropylene-based resins, for example, examples of the sheet materials include a nonwoven sheet made solely of propylene homopolymer (hereinafter homo PP) fiber, a nonwoven sheet made solely of random PP fiber having a small amount of ethylene randomly incorporated in the propylene chain, a nonwoven sheet made of sheath/core or side-by-side conjugate fiber containing at least 50% of a homo PP resin or a random PP resin and other thermoplastic resins (such as polyethylene resin or polyester resin), a nonwoven sheet made of blended fiber containing at least 50% of a homo PP resin or a random PP resin and other fiber (such as rayon fiber, cotton fiber, polyethylene fiber, or polyester fiber), and the like.

When the fusible components of the outer sheet 61 and the inner sheet 62 are polyethylene- or polyester-based resins, the term "of the same base" is interpreted in the same way as the polypropylene-based resin. When the fusible components are polyethylene-based resins, preferred examples of the sheet materials are nonwoven sheets made of blended fiber containing side-by-side or sheath/core conjugate fiber having a polyethylene resin as a sheath and a polyester resin as a core or side-by-side or sheath/core conjugate fiber having a polyethylene resin as a sheath and a polypropylene resin as a core, and the like. When the fusible components are polyester-based resins, preferred examples of the sheet materials are nonwoven sheets made of blended fiber containing side-by-side or sheath/core conjugate fiber having a low-melting polyester resin as a sheath and a polyester resin as a core, and the like. When the fusible components of the members making up the overlapped portion 7 are of the same base selected from polypropylene, polyethylene, and polyester, they exhibit good compatibility with each other to provide sufficient fusion bond strength so that the sheets may be fused together at lower temperatures. Since high temperatures are not needed in effecting embossing, the non-compressed regions are prevented from hardening by the embossing operation. As a result, there are formed side seals soft as a whole to provide improved feel to the touch.

It is preferred that all the sheets constituting the waist end region 71 of the overlapped portion 7, i.e., the outer sheet 61 (including the folded-over portion 61a) and the inner sheet 62 (including the folded-over portion 62a) of the diaper 1 be nonwoven fabric in terms of softness and cost.
The total basis weight of the sheets making up the waist end region 71 of the overlapped portion 7 of the diaper 1, i.e., the sum of the total basis weight of the outer sheet 61 and its folded-over portion 61a (i.e., twice the basis weight of the outer sheet 61) and the total basis weight of the inner sheet 62 and its folded-over portion 62a (i.e., twice the basis weight of the inner sheet 62) is preferably 120 g/m² or more, more preferably 130 g/m² or more, in terms of cushioning and improved feel to the touch. The upper limit of the total basis weight is preferably, but not limited to, 200 g/m², more preferably 180 g/m², from the economical viewpoint.

In the case of the diaper 101, it is preferred that both the outer sheet 61 (including the folded-over portion 61a) and the inner sheet 62 (including the folded-over portion 62a) be nonwoven fabric from the standpoint of softness and cost.
The total basis weight of the sheets making up the waist end region 181 of the side seal 108 of the diaper 101, i.e., the sum of the total basis weight of the outer sheet 61 and its folded-over portion 61a (i.e., twice the basis weight of the outer sheet 61) and the total basis weight of the inner sheet 62 and its folded-over portion 62a (i.e., twice the basis weight of the inner sheet 62) is preferably 120 g/m² or more, more preferably 130 g/m² or more, in terms of cushioning and improved feel to the touch. The upper limit of the total basis weight is preferably, but not limited to, 200 g/m², more preferably 180 g/m², from the economical viewpoint.

### Method of basis weight measurement:

Each of the inner sheet 62 and the outer sheet 61 from a diaper is cut to make a 30 mm by 30 mm specimen having a total area of 900 mm². The weight of the specimen is measured with an electronic balance having a minimum readability of 1 mg, and the weight is converted to a value on a per unit area basis. The basis weight measurement is taken on specimens from five diapers (n=5) to obtain an average. Note that the specimen each of the inner sheet 62 and the outer sheet 61 is cut out of a region other than the side seal 8 or 108. If a specimen is not obtained with the size of 30 mm by 30 mm, a specimen with as large an area as possible is cut out, and its weight is converted on a per unit area basis. In the case where the inner sheet and the outer sheet are bonded with an adhesive, such as a hot melt adhesive, the sheets are freed from the adhesive by washing with an organic solvent, e.g., chloroform, thoroughly dried, and then each weighed.

The topsheet 2, the backsheet 3, the absorbent member 4, and the sheet forming the side cuff 51 may be of any materials that have been commonly used in absorbent articles including disposable diapers. The topsheet 3 may be formed of liquid permeable nonwoven fabric, perforated film, or a laminate thereof. The backsheet 3 may be formed of resin film or a laminate composed of resin film and nonwoven fabric. The absorbent member 4 may be an aggregate of a fibrous material, e.g., pulp, or a combination of the fibrous material and a superabsorbent polymer, enclosed in an overwrap, such as tissue or water pervious nonwoven fabric. The sheet forming the side cuff 51 may be extensible film, nonwoven fabric, woven fabric, or a laminate thereof.

The side cuff elastic member 52, the waist elastic member 63, leg elastic member 64, and the below-waist elastic member 65 may be of any materials that have been commonly used in absorbent articles including disposable diapers. For example, elastic materials made of natural rubber, polyurethane, a styrene-isoprene copolymer, a styrene-butadiene copolymer, or an ethylene-α-olefin copolymer (e.g., ethyl acrylate-ethylene copolymer) may be used.

While the absorbent article of the invention has been described mainly with reference to the diapers 1 and 108, the pull-on absorbent articles of the invention are not limited to the diapers 1 and 108, and various changes and modifications may be made thereto.

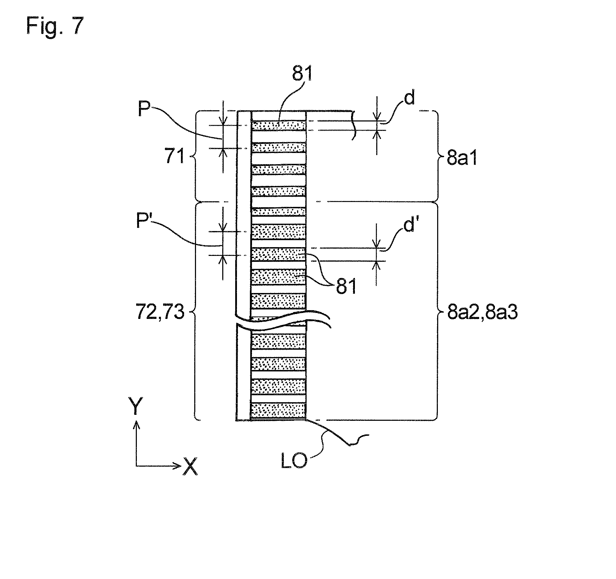

For instance, as shown in Fig. 7, the pitch P of the fusion bonds 81 in the seal region 8a1, which is formed by fusion bonding the waist end region 71, may be the same as the pitch P' in the seal regions 8a2 and 8a3, which are formed by fusion bonding the regions 72 and 73 below the waist end region 71, and instead, the length d of the fusion bonds 81 in the seal region 8a1 is made shorter than the length d' of the fusion bonds 81 in the seal regions 8a2 and 8a3, both in the longitudinal direction (Y-direction), whereby the total length D1 of the fusion bonds 81 in the seal region 8a1 is shorter than the total lengths D2 and D3 in the seal regions 8a2 and 8a3, respectively.

In that modification, the pitch P and P' of the fusion bonds 81 in the seal region 8a1 and the lower seal regions 8a2 and 8a3 is preferably 0.5 to 5.0 mm, more preferably 1 to 3 mm. The ratio of the length d of the fusion bonds in the seal region 8a1 to the length d' of the fusion bonds in the lower portions 8a2 and 8a3 (the former/the latter) is preferably 0.2 to 0.8, more preferably 0.4 to 0.7.

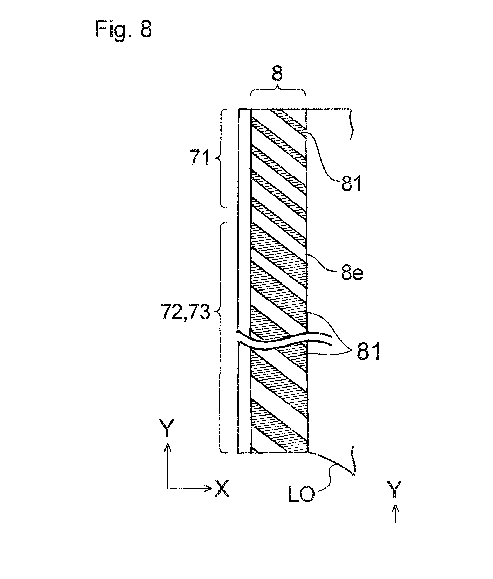

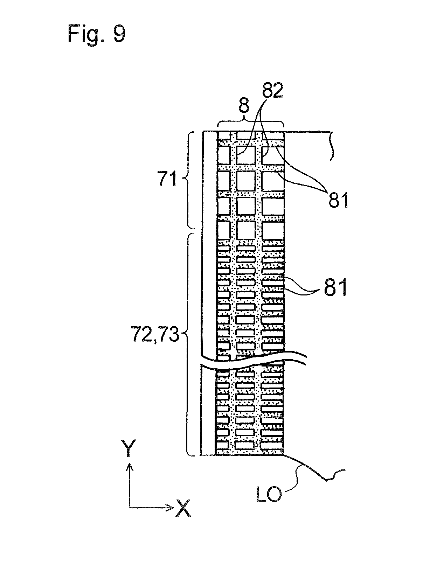

As shown in Fig. 8, the individual fusion bonds 81 of the side seal 8 may be oblique to the width direction of the side seal 8 (X-direction). The direction of obliquity may be the reverse of that shown in Fig. 8. As shown in Fig. 9, the fusion bonds may be formed in the side seal 8 in a lattice pattern composed of fusion bonds 81 extending in the width direction of the side seal 8 (X-direction) and fusion bonds 82 extending in the longitudinal direction of the side seal 8 (Y-direction) provided that the fusion bond 82 is absent along the lateral edges of the side seal 8.
In the modifications shown in Figs. 8 and 9, too, the same effects as obtained with the diaper 1 are produced by making the total length D1 of the fusion bonds 81 in the waist end region 71 shorter than the total lengths D2 and D3 of the fusion bonds 81 in the regions 72 and 73 below the waist end region 71.

While in the diaper 1 the outer sheet 61 and the inner sheet 62 are both folded back along the peripheral edge We of the waist opening WO of the front section A and the rear section B to make the waist end region 71 having a stack of eight sheets, the waist end region may have a stack of six sheets formed by folding only one of the outer sheet 61 and the inner sheet 62.
The region 72 having a stack of six sheets and the region 73 having a stack of four sheets, which are below the waist end region 71, may be replaced with either a region 72 having a stack of six sheets or a region 73 having a stack of four sheets.
While in the diaper 1 both the outer sheet 61 and the inner sheet 62 are folded over symmetrically between the front section A and the rear section B to form the folded-over portions (61a and 61b) and (62a and 62b), respectively, the sheets to be folded over or the length of the folded-over portions may be different between the front section A and the rear section B.

The side seal 8 does not have to be formed by a single pass through an embossing nip. For example, the side seal 8 may be formed by three embossing operations using an embossing roller having projections corresponding to the seal region 8a1 an embossing roller having projections corresponding to the seal region 8a2, and an embossing roller having projections corresponding to the seal region 8a3.

While in the diaper 101 the front side seal pattern on the side of the front section A is composed of the wider-than-long depressions shown in Fig. 12(a), and the rear side seal pattern on the side of the rear section B is composed of the longer-than-wide depressions shown in Fig. 12(c), the same effects of the diaper 101 will be obtained when the rear side seal pattern on the side of the rear section B is the pattern of Fig. 12(a), and the front side seal pattern on the side of the front section A is the pattern of Fig. 12(c).

One of the front side seal pattern and the rear side seal pattern may be composed of wider-than-long depressions 83 and 84 of Fig. 15(a), and the other may be composed of oblique depressions 86 shown in Fig. 15(b). The combination of these seal patterns provides the side seal 8 shown in Fig. 15(c), in which strongly bonded regions 93 are distributed in the planar direction of the side seal, and the number or the total area of the strongly bonded regions 93 in the waist opening-side end region 181 is smaller than that in the other region 182 so that the fusion bond strength of the end region 181 is lower than that of the other region 182. Thus, the side seal shown in Fig. 15(c) is effectively prevented from causing discomfort or irritation to the skin while retaining good cushioning and feel to the touch of the waist opening peripheral portion.

One of the front side seal pattern and the rear side seal pattern may be composed of oblique depressions 87 shown in Fig. 16(a), and the other may be composed of oblique depressions 88 shown in Fig. 16(b). The combination of these seal patterns provides the side seal 108 shown in Fig. 16(c), in which strongly bonded regions 93 are distributed in the planar direction of the side seal, and the number or the total area of the strongly bonded regions 93 in the end region 181 on the waist opening side is smaller than that in the other region 182 so that the fusion bond strength of the end region 181 is lower than that of the other region 182. Thus, the side seal shown in Fig. 16(c) is effectively prevented from causing discomfort or irritation to the skin as well while retaining good cushioning and feel to the touch of the waist opening peripheral portion.

In the case where the width W1 of the oblique depressions or wider-than-long depressions formed in the waist opening-side end region 181 and the width W2 of the oblique depressions or wider-than-long depressions formed in the other region 182 are different as shown in Fig. (16a), the ratio of the width W1 to the width W2 (W1/W2) may be, for example 0.2 to 0.8, preferably 0.3 to 0.5. The width W1 indicated in Figs. 15(b), 16(a), and 16(b) is preferably in the same range as set forth for the length d' of the wider-than-long depressions 83 in the diaper 101.

While in the diaper 101 the outer sheet 61 and the inner sheet 62 are both folded back along the peripheral edge We of the waist opening WO of the front section A and the rear section B, it may be only one of the outer sheet 61 and the inner sheet 62 that is folded back.
Instead of providing, below the end region 181, one region 182 of which the seal pattern is different from the seal pattern of the end region 181, two or more regions 182 each having a different seal pattern from the seal pattern of the end region 181 may be provided below the end region 181. In this modification, too, the end region 181 should have the highest fusion bond strength.
While in the diaper 101 both the outer sheet 61 and the inner sheet 62 are folded over symmetrically between the front section A and the rear section B to form the folded-over portions (61a and 61b) and (62a and 62b), respectively, the sheets to be folded over or the length of the folded-over portions may be different between the front section A and the rear section B.

The pull-on absorbent articles according to the invention include not only disposable pull-on diapers but other pull-on absorbent articles, such as pant-type sanitary napkins.

With respect to particulars that have not been described for one embodiment, the corresponding details of other embodiments appropriately apply, and the particulars described as being characteristic of one embodiment appropriately apply to other embodiments. Particulars of each of the aforementioned embodiments are appropriately interchangeable with each other.

### Industrial Applicability

The pull-on absorbent article of the invention hardly causes discomfort or irritation to the skin in the side seals thereof, exhibits good cushioning and feel to the touch in the waist portion thereof, and provides excellent wearer comfort.

## Claims

1. A pull-on absorbent article comprising a front section having lateral side edge portions and a rear section having lateral side edge portions, the lateral side edge portions of the front section and the lateral side edge portions of the rear section being overlapped to form overlapped portions, and the overlapped portions being fusion bonded to form a pair of side seals,
the overlapped portions each having a waist end region on the side of a waist opening, the waist end region comprising a stack of larger number of sheets than any other region of the overlapped portion,
the stack of the sheets forming the waist end region in both the front section and the rear section extending over a whole distance between opposing overlapped portions of the front section and between opposing overlapped portions of the rear section, respectively, and
the side seal having a smaller total length of fusion bonds per unit length in its longitudinal direction measured along a skin side edge in a seal region formed by fusion bonding the waist end region than in any other seal region formed by fusion bonding an other region below the waist end region.

2. The pull-on absorbent article according to claim 1, comprising a oblong absorbent assembly having a topsheet and a absorbent member and an outer cover located on a garment facing side of the absorbent assembly to fix the absorbent assembly,
the outer cover comprising an outer sheet defining the exterior surface of the pull-on absorbent article and an inner sheet overlaid on an inner side of the outer sheet, and
the outer cover forming the overlapped portions.

3. The pull-on absorbent article according to claim 2, wherein the outer sheet and/or the inner sheet is folded over along a line located outward of a longitudinal ends of the absorbent assembly onto the inner sheet to provide a folded-over portion, and the waist end region is a region of the overlapped portion that contains the folded-over portion of the outer sheet and/or the inner sheet.

4. The pull-on absorbent article according to any one of claims 1 to 3, wherein the side seal has a lower fusion bond strength in the waist end region than in the other region below the waist end region.

5. The pull-on absorbent article according to any one of claims 1 to 4, wherein
the side seal has a front side seal pattern formed by pressing a side of the front section and a rear side seal pattern formed by pressing a side of the rear section and comprises an end region on the side of the waist opening and other region,
the front side seal pattern and the rear side seal pattern being different from each other, the front side seal pattern or the rear side seal pattern being different between the end region and the other region, and
the side seal having a lower fusion bond strength in the end region than in the other region.

6. The pull-on absorbent article according to claim 5, wherein an overlap between the front side seal pattern and the rear side seal pattern is absent along the skin side edge of the side seal.

7. The pull-on absorbent article according to claim 5 or 6, wherein the end region having a different seal pattern from the other region is in a region of the overlapped portion that contains the largest number of the sheets stacked.
